# DEMANDE DE BREVET EUROPEEN

(11) **EP 0 606 188 A1**
(43) Date de publication de la demande: **13.07.1994**
(21) Numéro de dépôt: 94400044.7
(22) Date de dépôt: 07.01.1994
(51) Int. Cl.: A61F 9/00

(54) **Bouchon méatique pour phathologie lacrymale**

(30) Priorité: 08.01.1993 FR 9300122
(71) Demandeur: F.C.I.(FRANCE CHIRURGIE INSTRUMENTATION) S.A., F-92130 Issy-Les Moulineaux (FR)
(72) Inventeur: Bernard, Jean-Antoine, F-75016 Paris (FR)
(74) Mandataire: Viard, Jean

(57) **Abrégé**

- Bouchon méatique pour pathologie lacrymale comprenant une collerette, un col et un bulbe. La partie centrale du bulbe (1) est évidée pour former une aile élastique (5) donnant de l'élasticité au bouchon lors de son insertion et ancrant celui-ci au-dessous du méat afin d'éviter des expulsions accidentelles.

## Description

La présente invention a pour objet un bouchon ou clou méatique pour pathologie lacrymale destiné à obturer les canalicules lacrymaux en particulier, mais non exclusivement, en ophtalmologie à remédier aux kérato-conjonctivites sèches, maladies désignées couramment par "yeux secs". Une telle maladie peut être due soit à une génération insuffisante de liquide lacrymal, soit à un drainage excessif de celui-ci par les canalicules permettant normalement l'évacuation des larmes à l'intérieur de la cavité nasale.

On a déjà proposé dans US-A-3 949 750 de remédier temporairement à un tel phénomène en obturant les méats des canalicules au moyen d'un "clou" ou "bouchon". les bouchons connus comprennent une partie médiane ou col, relativement longue d'un diamètre inférieur à ceux des deux parties d'extrémités: collerette et bulbe, la paroi interne du méat venant s'appliquer contre la partie médiane en formant un joint d'étanchéité. Le bouchon est ainsi maintenu dans le méat et ferme l'ouverture supérieure de celui-ci à l'aide d'une pièce pleine. L'extrémité inférieure du bouchon présente une forme de cône ou bulbe permettant une meilleure introduction du bouchon dans le méat.

Dans FR-A- 2 644 058, il a été proposé d'incliner la partie supérieure du bouchon ou collerette pour éviter le frottement du bord de celle-ci contre la cornée ce qui provoque des irritations. Par ailleurs, ce document propose de percer le bouchon par un canal axial en vue de traiter I'atrésie.

FR-A- 2 641 692 décrit plusieurs formes de bouchons pour colmater des brèches diverses dans certains desquels au moins une partie est élastique. Lors de l'insertion dans la brèche, le bouchon peut être déformé pour faciliter l'opération et reprendre ensuite sa forme d'origine.

Les bouchons de l'art antérieur présentent cependant des inconvénients. Lors de l'insertion, le méat doit être ouvert au diamètre le plus grand du cône or, le bouchon est souple, d'où des difficultés. Par ailleurs, on a pu observer des expulsions accidentelles lors du port de tels bouchons. Ces expulsions sont dues au fait que les bulbes des bouchons connus sont coniques et pleins, leur pointe étant orientée vers le bas. Dans ces conditions, la surface d'appui du bulbe contre la surface inférieure de la paroi du méat est plane et cette surface peut glisser sur la paroi ce qui se traduit par un rejet, l'organisme ayant tendance à repousser tout ce qui lui est étranger.

La présente invention a pour objet de pallier ces inconvénients.

Selon la présente invention, le bouchon méatique pour pathologie lacrymale comprenant de bas en haut: un bulbe, un col et une collerette, est caractérisé en ce que le bulbe présente une partie centrale évidée autour du col, délimitée par une aile souple périphérique.

La partie supérieure du bulbe forme une aile circulaire souple qui permet un véritable ancrage, dans le canalicule, au-dessous du méat. L'expulsion du bouchon est ainsi évitée, bien que l'extraction volontaire reste toujours possible. Par ailleurs, les bouchons sont fabriqués en silicone et présentent par suite une bonne souplesse. L'aile du bulbe se comprime tout naturellement vers le centre par contact avec la paroi du méat lors de l'insertion.

D'autres caractéristiques et avantages de l'invention apparaîtront au cours de la description qui va suivre , d'un mode particulier de réalisation donné à titre d'exemple non limitatif, en regard des figures qui représentent :
- la figure 1, une vue en perspective d'un bouchon selon l'invention;
- la figure 2, une vue schématique de l'insertion d'un bouchon dans un canalicule;
- la figure 3, une vue de l'implantation d'un bouchon dans le canalicule.

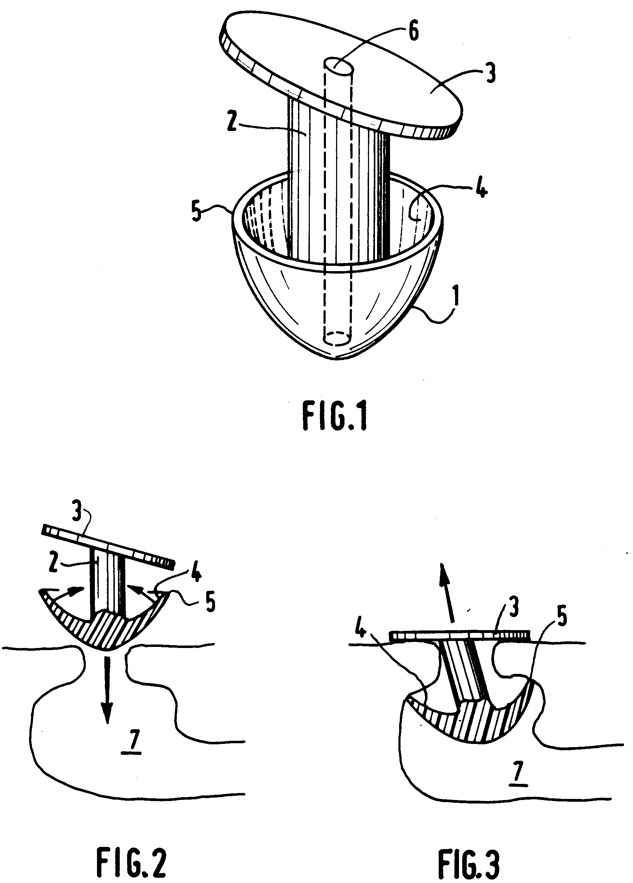

Sur la figure 1, on distingue le bulbe 1, le col 2 et la collerette 3 qui, dans l'exemple représenté est inclinée par rapport à l'axe longitudinal du bouchon. Le bouchon présente également un trou axial borgne 6 qui a pour but de permettre l'insertion du bouchon dans le méat au moyen d'un instrument spécial appelé "pose-bouchon" ou "pose clou" qui comprend , à l'intérieur d'une gaine une tige rigide pouvant s'insérer dans le trou 6 . Lors de l'insertion, la tige (non représentée) fait saillie dans le trou 6 jusqu'à atteindre le fond de celui-ci pour pouvoir entraîner le bouchon dans le méat alors que le noyau central du bouchon est rigidifié. Après insertion du bouchon dans le méat, la tige est rétractée à l'intérieur de la gaine et le bouchon retrouve sa souplesse d'origine. A cet effet, et selon une caractéristique de l'invention, le fond du trou 6 se trouve au-dessous du niveau de la partie supérieure du bulbe 1 de manière à ce que la tige rigidifie la partie centrale du bouchon sur toute sa hauteur.

La partie centrale supérieure du bulbe 1 présente un évidement 4 délimité, à sa périphérie, par une couronne ou aile circulaire 5. ce qui lui donne une forme de champignon ou parapluie retourné. Comme indiqué précédemment, le bouchon est réalisé en silicone et l'amincissement effectué entre le pied du bulbe et l'aile 5 se traduit par une grande élasticité de celle-ci en direction radiale vers le centre. Cette élasticité est mise à profit pour faciliter l'insertion du bouchon dans le méat 7 comme cela apparaît sur la figure 2. Lorsqu'une pression est exercée dans le sens de la flèche F1, l'aile se rabat vers le centre sous l'action de la paroi du méat 7 comme schématisé par les flèches F3.

L'insertion est ainsi doublement facilitée par la rigidité de la partie centrale ou noyau du bouchon et par la compressibilité du bulbe.

Par contre, comme cela apparaît sur la figure 3, après passage du méat 7, l'aile 5 se détend, par élasticité de sorte que le bord de l'aile 5 vienne prendre appui contre la surface inférieure 8 du méat. L'élasticité de l'aile 5 étant dans le sens opposé, il en résulte que le bouchon est en quelque sorte ancré à la manière d'un grappin contre la paroi interne du méat qui est constituée par un tissu résistant à l'encontre de la force d'expulsion symbolisée par la flèche F2.

Un bouchon du type qui vient d'être décrit peut, avantageusement être utilisé pour constituer la tête d'une sonde monocanaliculaire, la sonde faisant saillie latéralement à partir du bulbe.

Il est également possible d'utiliser un bulbe tel que ci-dessus dans le cas d'un clou-trou, c'est à dire d'un bouchon à trou central débouchant en vue de traiter l'atrésie.

## Revendications

**1°** Bouchon méatique pour pathologie lacrymale comprenant de bas en haut: un bulbe (1), un col (2) et une collerette (3) , caractérisé en ce que le bulbe présente une partie centrale évidée (4) autour du col (2) délimitée par une aile souple périphérique (5).

**2°** Bouchon méatique selon la revendication 1, caractérisé en ce qu'il présente un orifice axial (6) s'étendant depuis la collerette jusqu'à un niveau inférieur à celui de la partie supérieure du bulbe (1).

**3°** Bouchon méatique selon la revendication 1, caractérisé en ce qu'il présente un orifice axial débouchant

**4°** Sonde monocanaliculaire, caractérisée en ce que son extrémité consiste en un bouchon selon la revendication 1.
